(19) Europäisches Patentamt
European Patent Office
Office européen des brevets

(11) Numéro de publication : **0 422 978 B1**

# (12) FASCICULE DE BREVET EUROPEEN

(45) Date de publication du fascicule du brevet : **22.12.93 Bulletin 93/51**

(51) Int. Cl.$^5$ : **A61K 9/127**

(21) Numéro de dépôt : **90402648.1**

(22) Date de dépôt : **26.09.90**

## (54) Procédé de fabrication d'une dispersion aqueuse de vésicules lipidiques et composition à base de la dispersion ainsi obtenue.

(30) Priorité : **12.10.89 FR 8913358**

(43) Date de publication de la demande : **17.04.91 Bulletin 91/16**

(45) Mention de la délivrance du brevet : **22.12.93 Bulletin 93/51**

(84) Etats contractants désignés : **AT BE CH DE DK ES FR GB GR IT LI NL SE**

(56) Documents cités : **EP-A- 0 055 576**

(73) Titulaire : **L'OREAL
14, Rue Royale
F-75008 Paris (FR)**

(72) Inventeur : **Grollier, Jean-François
16 Bis, boulevard Morland
F-75004 Paris (FR)**
Inventeur : **Rosenbaum, Georges
2, rue J. H. Mansart
F-92600 Asnieres (FR)**
Inventeur : **Hansenne-Richoux, Isabelle
19, rue Boursault
F-75017 Paris (FR)**
Inventeur : **Chiodi, Francisco
35, rue de Boulainvilliers
F-75016 Paris (FR)**
Inventeur : **Burin, Jacky
38, allée Jean Giono
F-93270 Sevran (FR)**

(74) Mandataire : **Peuscet, Jacques et al
Cabinet Peuscet 68, rue d'Hauteville
F-75010 Paris (FR)**

**EP 0 422 978 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention porte sur un procédé de fabrication de dispersions aqueuses de vésicules lipidiques, ainsi que sur les compositions à base des dispersions ainsi obtenues.

Les vésicules lipidiques de ces dispersions ont une structure lamellaire constituée d'au moins un feuillet formé d'une bi-couche lipidique définissant un volume fermé ; ils encapsulent une phase aqueuse comprenant avantageusement des substances actives hydrosolubles, par exemple pharmaceutiques ou cosmétiques, lesquelles sont ainsi protégées des conditions extérieures. Ces vésicules peuvent être réalisées à partir de lipides ioniques ou de lipides non-ioniques : le terme "vésicule" utilisé dans la présente demande englobe les deux types de structure.

On connaît, par la demande de brevet français FR-A-2 399 242, un procédé de préparation de liposomes en suspension aqueuse, suivant lequel : on disperse une première phase aqueuse pouvant contenir des actifs dans un solvant non miscible ou peu miscible à l'eau, en présence d'un lipide de formule XY, où X est un groupe hydrophile et Y est un groupe lipophile, ce qui permet d'obtenir dans une phase solvant continue des "précurseurs de liposomes" consistant en des globules microscopiques de phase aqueuse, dont l'enveloppe est une pellicule de la substance XY ; on émulsifie ces "précurseurs de vésicules dans un milieu aqueux en présence d'un excès du lipide XY ou d'un autre lipide ZW, où Z est un groupe hydrophile et W est un groupe lipophile, le solvant précité étant éliminé avant ou après ladite émulsification. Cependant, ce procédé requiert d'utiliser des vibrations soniques ou ultrasoniques pour la dispersion de la première étape, technique mal adaptée aux productions à l'échelle industrielle.

Par le brevet français FR-B-2 418 023, on connaît un procédé de fabrication de capsules synthétiques oligolamellaires lipidiques propres à renfermer un (ou des) matériau(x) biologiquement actif(s) suivant lequel : on prépare un mélange d'une phase organique contenant, dans un solvant, un composé formateur de parois des capsules et d'une phase aqueuse contenant le matériau biologiquement actif destiné à être encapsulé, le rapport de la phase organique à la phase aqueuse étant d'une valeur propre à fournir une émulsion du type eau-dans-huile (E/H) ; on forme une émulsion homogène de ce type ; on évapore le solvant organique de l'émulsion jusqu'à ce qu'on obtienne un mélange ayant le caractère d'un gel ; et on transforme ce mélange en une suspension de capsules oligolamellaires synthétiques renfermant le matériau biologiquement actif en le dispersant dans un milieu aqueux. Dans ce procédé, la concentration des lipides dans la phase organique doit cependant rester relativement faible : on indique une gamme allant de 0,05 à 5% en poids, ce qui ne permet pas d'obtenir facilement des compositions à forte concentration de vésicules. Par ailleurs, l'émulsification est obtenue de préférence, par ultrasons, ce qui n'est guère applicable à l'échelle industrielle.

Dans la demande de brevet japonais 87/273386 (publiée sous le n° 1117824),on a décrit un procédé de préparation de vésicules dans lequel on dissout des lipides dans un solvant organique, on ajoute de l'eau, on élimine le solvant pour former une phase hydratée et on disperse cette phase hydratée dans une phase aqueuse pour former des vésicules. Ce procédé nécessite un traitement de la dispersion obtenue pour réduire la taille des vésicules et la dispersion des tailles, ce traitement mettant en oeuvre soit des ultrasons, soit un homogénéisateur. L'inconvénient des ultrasons a déjà été explicité ; celui d'une homogénéisation, outre le fait qu'elle élève la température de dispersion, ce qui peut être gênant pour des actifs encapsulés thermosensibles, est de libérer des particules métalliques dans le milieu, ce qui peut être néfaste pour des actifs encapsulés sensibles à des traces de métaux.

La demande de brevet français FR-A-2 561 101 décrit un procédé de préparation de liposomes consistant à former une émulsion de type E/H par dispersion d'une première phase aqueuse dans un solvant organique non miscible à l'eau de présence de molécules comportant une fraction lipophile et une fraction hydrophile, puis à éliminer la majeure partie du solvant organique de cette émulsion E/H par une méthode douce, pour obtenir une émulsion E/H concentrée, et à ajouter progressivement à cette émulsion, sous agitation, une seconde phase aqueuse contenant des molécules comportant une fraction lipophile et une fraction hydrophile, en éliminant simultanément le solvant organique résiduel par évaporation. Dans ce cas également, la concentration en lipides dans la phase organique est faible : on a indiqué, à cet égard, une gamme allant de 0,5 à 5% en poids. De plus, l'élimination du solvant organique de la deuxième étape est effectuée principalement par décantation, ce qui demande beaucoup de temps et nuit, de ce fait, à l'application industrielle de ce procédé.

On connaît enfin, par la demande de brevet européen EP-A-349 429, un procédé de préparation de systèmes colloïdaux dispersibles de lipides amphiphiles, sous forme de liposomes oligolamellaires submicroniques, dont la paroi est constituée par lesdits lipides et éventuellement une substance A et dont le noyau est constitué par de l'eau ou une solution aqueuse contenant éventuellement une substance B. Selon ce procédé, on prépare une phase liquide constituée essentiellement par une solution des lipides (et éventuellement de la substance A) dans un solvant pouvant contenir la substance B en solution ; on prépare une seconde phase liquide constituée essentiellement par de l'eau ou une solution aqueuse de la substance B ; on ajoute, sous

agitation modérée, la première phase à la seconde phase, de manière à obtenir pratiquement instantanément, une suspension colloïdale de vésicules : si on le désire, on élimine tout ou partie du solvant et de l'eau, de manière à obtenir une suspension colloïdale de concentration voulue en vésicules. En pratique, les solvants utilisés sont des solvants miscibles à l'eau. On a indiqué que la concentration en lipides dans le solvant va de 0.1 à 10 % en poids, ce qui est très limité.

La présente invention vise à proposer un procédé de préparation de suspensions aqueuses de sphérules lipidiques bien adapté aux applications industrielles. Cet objectif est atteint, conformément à l'invention, par l'utilisation d'un solvant non miscible à l'eau pour dissoudre les lipides devant constituer les feuillets des vésicules et par la dispersion dans une phase aqueuse ce cette phase de solvant ayant dissout les lipides pour obtenir une émulsion huile-dans-eau (H/E) avec mise en oeuvre d'une agitation pour réaliser cette dispersion. Le procédé selon l'invention offre l'avantage de permettre d'opérer à basse température puisqu'il n'est pas nécessaire de fondre les lipides qui sont dissouts dans la phase solvant, ce qui, en particulier, autorise l'encapsulation dans les vésicules de substances actives thermo-sensibles ; il permet aussi d'utiliser une phase organique concentrée. Mais surtout, et de façon tout à fait inattendue et inexpliquée, il aboutit toujours directement à une phase vésiculaire de très bonne qualité, c'est-à-dire à des vésicules de faible dispersité et de forts taux d'encapsulation, ce qui dispense d'une étape ultérieure d'homogénéisation et évite donc tous les inconvénients susmentionnés d'une telle étape.

Par ailleurs, le procédé selon l'invention est plus rapide que le procédé classique consistant à mettre en contact les lipides avec la phase aqueuse à encapsuler dans les vésicules, à agiter doucement pour former une phase lamellaire, à ajouter un liquide de dispersion, puis à agiter énergiquement ; en effet, on supprime l'étape d'hydratation pour la formation de la phase lamellaire, étape qui était toujours assez longue.

La présente invention a donc d'abord pour objet un procédé de préparation d'une dispersion aqueuse de vésicules lipidiques lamellaires, chacune de ces vésicules étant constituée par au moins une feuillet lipidique sphéroïde encapsulant un liquide aqueux, dans lequel :

(a) dans une première étape, on dissout au moins un lipide dans au moins un solvant organique,

(b) dans une deuxième étape, on ajoute la phase organique obtenue à la première étape à une phase aqueuse,

(c) dans une troisième étape, on disperse le mélange de la deuxième étape sous agitation, et

(d) dans une quatrième étape, on évapore le (ou les) solvant (s) en même temps qu'une partie de l'eau,

caractérisé par le fait que le (s) solvant (s) organiques (s) de l'étape a) est (sont) non miscible(s) à l'eau ; que les quantités respectives des deux phases de l'étape b) sont telles que la dispersion obtenue ultérieurement soit une dispersion du type huile-dans-eau (H/E) ; et que les étapes c) et d) sont menées sous forte agitation, la phase continue de la dispersion restant en permanence la phase aqueuse.

Dans ce procédé, le cas échéant, dans une cinquième étape, on concentre la dispersion.

A l'étape (a), on peut utiliser avantageusement tous les solvants non-miscibles à l'eau qui ont un point d'ébullition inférieur à 50°C à une pression comprise entre 1,5 x $10^4$ et $10^5$ Pa et solubilisent les lipides utilisés pour former les vésicules. Comme solvants utilisables conformément à l'invention, on peut citer le dichlorométhane, le chloroforme, l'acétate d'éthyle, l'acétate de butyle, le formiate d'éthyle, l'hexane, le cyclohexane, le toluène, l'éther de pétrole, et les mélanges d'au moins deux d'entre eux.

On peut ainsi préparer à l'étape (a) une phase organique dans laquelle le (ou les) lipide (s) est (ou sont) présent(s) à raison d'environ 5 à 50 % en poids, de préférence, d'environ 10 à 20 % en poids de ladite phase organique. Cette haute concentration en lipides est une caractéristique très intéressante du procédé selon l'invention.

Par ailleurs, l'étape (a) du procédé selon l'invention est avantageusement conduite à une température d'environ 35°C à environ 55°C, de préférence, à une température se situant aux environs de 35°C.

On peut choisir le (ou les) lipide(s) parmi les lipides amphiphiles non-ioniques, les lipides amphiphiles ioniques, et les mélanges de lipides non-ionique(s) et ionique(s).

Les lipides amphiphiles non-ioniques sont notamment choisis parmi :

(1) les éthers de polyglycérol, linéaires ou ramifiés, de formule :

$$R\,O\!\left[C_3H_5(OH)O\right]_{\overline{n}}\,H$$

dans laquelle :

. $-C_3H_5(OH)O$ est représenté par les structures suivantes prises en mélange ou séparément :
$-CH_2CHOHCH_2O-$ ;

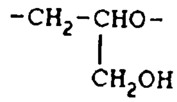 ; 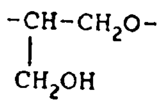 ;

. $\bar{n}$ est une valeur statistique moyenne comprise entre 2 et 6 ;

. R représente :

(a) une chaîne aliphatique, linéaire ou ramifiée, saturée ou insaturée, contenant de 12 à 30 atomes de carbone ; ou des radicaux hydrocarbonés des alcools de lanoline ;

(b) un reste $R^1CO$, où $R^1$ est un radical aliphatique, linéaire ou ramifié, en $C_{11}$-$C_{17}$ ;

(c) un reste $R^2\{OC_2H_3(R^3)\}$, où :

. $R^2$ peut prendre la signification (a) ou (b) donnée pour R ;

. $OC_2H_3(R^3)$- est représenté par les structures suivantes, prises en mélange ou séparément :

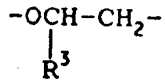 et 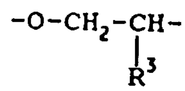

où $R^3$ prend la signification (a) donnée pour R ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétique ;

(7) les hydroxyamides représentés par la formule :

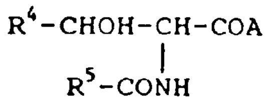

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :

. un reste

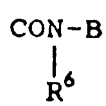

où :

. B est un radical dérivé d'amines primaires ou secondaires, mono- ou polyhydroxylées ; et

. $R^6$ désigne un atome d'hydrogène ou un radical méthyle, éthyle ou hydroxyéthyle ; et

. un reste -COOZ, où Z représente le reste d'un polyol en $C_3$-$C_7$.

Les lipides amphiphiles ioniques sont notamment choisis parmi :

- les phospholipides naturels, tels que la lécithine d'oeuf ou de soja et la sphingomyéline ;
- les phospholipides de synthèse, tels que la dipalmitoyl-phosphatidylcholine ou la lécithine hydrogénée ; et
- les composés anioniques.

L'évaporation du solvant à l'étape (d) peut avantageusement être conduite à la pression atmosphérique. L'éventuelle concentration de la dispersion à la fin du procédé s'effectue par entraînement, sous pression réduite, du solvant résiduel éventuel et d'une partie de l'eau.

On peut associer aux lipides au moins un additif choisi parmi :

- les alcools et diols à longue chaîne ;
- les stérols, par exemple le cholestérol ;

- les amines à longue chaîne et leurs dérivés ammonium-quaternaires ;
- les dihydroxyalkyl-amines ; les amines grasses polyoxyéthylénées ; les esters d'amino-alcools à longue chaîne ; et leurs sels et dérivés ammonium-quaternaires ;
- les esters phosphoriques d'alcools gras, par exemple, le dicétylphosphate acide ou son sel de sodium ;
- les alkylsulfates, par exemple le cétylsulfate de sodium ; et
- certains polymères, tels que les polypeptides et les protéines.

Conformément à une autre caractéristique particulière de l'invention, à l'étape (b), on utilise un rapport pondéral de la phase organique à la phase aqueuse compris entre environ 0,1 et environ 0,6, conduisant à une émulsion H/E.

Par ailleurs, il va de soi que l'on peut incorporer au moins une substance active hydrosoluble à la phase aqueuse et/ou au moins une substance active liposoluble à la phase organique. On peut ainsi encapsuler des actifs hydrosolubles dans la phase aqueuse encapsulée dans les vésicules et des actifs liposolubles dans les feuillets des vésicules.

En outre, dans le procédé selon l'invention, il est important que le mélange de la phase organique contenant les lipides et de la phase aqueuse soit effectué sous une forte agitation. On peut notamment conduire les agitations des étapes (c) et (d) avec des organes d'agitation rotatifs dont la vitesse périphérique est supérieure à 10 m/s à l'extrémité libre desdits organes et dont la vitesse angulaire est supérieure à 1000 tours/mn.

Les vitesses préférées sont respectivement de 40 m/s avec 5000 tours/mn.

Selon l'invention, on peut régler la taille des vésicules en faisant varier la vitesse d'agitation au cours du mélange des phases. Cette taille peut être réglée, en particulier, entre 0,2 et 0,3 µm.

La présente invention porte également sur les compositions à base des dispersions aqueuses de vésicules lipidiques, telles qu'obtenues par le procédé qui vient d'être décrit.

Pour mieux faire comprendre l'objet de l'invention, on va en décrire maintenant, à titre d'exemple purement illustratif et non limitatif, un mode de mise en oeuvre.

<u>EXEMPLE 1</u> : Préparation d'une dispersion aqueuse de vésicules à partir de lipides non ioniques.

On utilise une phase lipidique formulée comme suit (% en poids) :

. Lipide amphiphile non-ionique de formule :

$$RO[C_3H_5(OH)O]_{\overline{n}}H$$

dans laquelle :

. R est un radical hexadécyle ;

. $-C_3H_5(OH)O$ représenté :

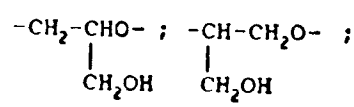

. $\overline{n}$ vaut 3 47,5

. Cholestérol 47,5

. Dicétyl phosphate 5

On prépare des vésicules lipidiques encapsulant une phase aqueuse à partir de la formulation suivant (% en poids) :

| | | |
|---|---|---|
| Phase lipidique ci-dessus définie | | 8,7 |
| Dichlorométhane | ............... | 34,8 |
| Phase aqueuse | Glucose ....... | 2 |
| | Glycérine ..... | 2,2 |
| | Eau ........... | 52,3 |
| | Conservateur .. | Quantité suffisante |

On dissout la phase lipidique dans le dichlorométhane à une température de 35°C ; puis on additionne la phase organique ainsi obtenue à la phase aqueuse et on disperse pendant 10 mn, sous une agitation à 5 000 tours/mn, avec une turbine donnant une vitesse périphérique en bout de pales de 40 m/s, dans un récipient

de 50 $dm^3$ de volume utile et d'environ 40 cm de hauteur contenant 20 kg de mélange.

On distille, sous la même agitation, le dichlorométhane, vers 40°C à la pression atmosphérique, pendant 55 minutes.

Ensuite, on réalise un entraînement du dichlorométhane résiduel et d'une partie de l'eau, vers 40°C, pendant 145 minutes en poursuivant la distillation sous une pression réduite de 4 x $10^3$ pascals.

On obtient une dispersion vésiculaire ayant une concentration pondérale en phase lipidique de 23,1 %. On observe que le taux d'encapsulation des vésicules à la fin de l'opération est de 2,4 µl/mg avec une fraction volumique de 79 % (la fraction volumique est le rapport du volume occupé par les vésicules au volume total de la dispersion) et un diamètre moyen de 200 nm. A titre de comparaison, il faut noter que les procédés courants conduisent à des vésicules de taille supérieure à 1000 nm. Pour les affiner il faut utiliser des homogénéisateurs classiques, à concentration lipidique équivalente, il faut alors plusieurs passages pour atteindre des vésicules d'environ 220 nm et, dans ce cas, le taux d'encapsulation ne dépasse pas 2 µl/mg pour des fractions volumiques inférieurs à 70 %.

L'évolution de la dispersion au cours de ce procédé a été suivie ; les résultats sont rapportés dans le tableau 1 ; le temps t= 0 correspond au début de distillation.

L'évolution de la conductance de la dispersion au cours du procédé a été mesurée à l'aide d'un galvanomètre associé à une électrode constituée de deux fils de platine distants de 0,5 cm et soumis à une différence de potentiel de 4 volts.

Les résultats sont portés sur la figure 1 du dessin annexé : le temps, en minutes, est porté en abscisse, et la conductance, en µSiemens, en ordonnée. On peut constater que la conductance se maintient entre 10 et 40 µSiemens : une variation d'une si faible amplitude est incompatible avec une inversion de phase ; le milieu continu de la dispersion (phase aqueuse) ne change donc pas de nature au cours de l'élimination du solvant.

TABLEAU 1

| Temps (mn) | Teneur $H_2O$ (%) | Teneur $CH_2Cl_2$ (%) | Conc. glucose (mol/l) | Conc. phase lipidique (%) | Fract.volumique (%) ** | Taux encapsul. (µl/mg) | Diam.moyen (nm) * | Polydispersité |
|---|---|---|---|---|---|---|---|---|
| 30 | --- | 25,7 | --- | --- | --- | --- | --- | --- |
| 35 | 72,2 | 20,9 | 0,126 | 4,6 | 35,8 | --- | --- | --- |
| 38 | 72,9 | 17,5 | 0,179 | 6,5 | 55,1 | --- | --- | --- |
| 45 | 76,8 | 11,1 | 0,207 | 8,1 | 67,9 | --- | --- | --- |
| 48 | 81,4 | 7,4 | 0,181 | 7,5 | 62,8 | --- | 347 | 0,64 |
| 55 | 79,4 | 2,7 | 0,296 | 12,0 | 74,3 | --- | 229 | 0,26 |
| 60 | 79,4 | ~0 | 0,341 | 13,9 | 76,7 | 4,5 | 219 | 0,20 |
| 110 | 78,5 | " | 0,360 | 14,5 | 76,0 | 4,3 | 218 | 0,19 |
| 120 | 76,3 | " | 0,408 | 15,9 | --- | --- | --- | --- |
| 130 | 72,8 | " | 0,491 | 18,3 | --- | --- | --- | --- |
| 137 | 69,8 | " | 0,568 | 20,3 | --- | --- | --- | --- |
| 145 | 65,6 | " | 0,689 | 23,1 | 79,2 | 2,4 | 221 | 0,19 |

* la granulométrie de la dispersion a été étudiée à l'aide du compteur "COULTER N4" après dilution dans le glucose 0,3 M + $NaN_3$ 0,2°/oo.

** fraction volumique totale de la phase dispersée (vésicules + phase organique) (lorsque la quasi-totalité du dichlorométhane a été éliminée, la fraction volumique totale est pratiquement égale à la fraction volumique des vésicules).

EXEMPLE 2 : Préparation d'une dispersion aqueuse de vésicules à partir de lipides non-ioniques

On utilise une phase lipidique formulée comme suit :

- Lipide non-ionique de formule :

$$C_{12}H_{25}[O\ C_2H_3\ (R)]\text{-}O\text{-}[C_3H_5(OH)\text{-}O]_{\bar{n}}\text{-}H$$

où $-O\ C_2H_3(R)-$ est constitué par un mélange des radicaux :

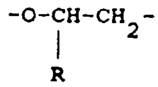 et 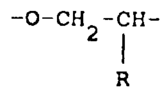 ;

où $-C_3H_5(OH)-O-$ est constitué par un mélange des radicaux :

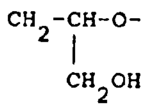 et 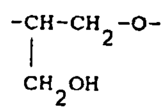 ;

où $\bar{n} = 6$ ;

et où R est un mélange des radicaux $C_{14}H_{29}$ et $C_{16}H_{33}$ 38O g

- Cholestérol 38O g
- Acide tout-trans rétinoïque 10 g
- DL-α-tocophérol 10g

Dans un bac cylindrique de 50 $dm^3$ de volume utile et d'environ 40 cm de hauteur, on dissout la phase lipidique dans 4000g de dichlorométhane à une température de 35°C ; puis on additionne la phase organique ainsi obtenue à 13855g d'eau et on disperse pendant 10 minutes sous une agitation à 5000 tours/mn avec une turbine donnant une vitesse périphérique en bout de pales de 40 m/s.

On distille, sous la même agitation, le dichlorométhane, vers 40°C à la pression atmosphérique, pendant 55 minutes.

Ensuite, on réalise un entraînement du dichlorométhane résiduel et d'une partie de l'eau, vers 40°C, pendant 145 minutes en poursuivant la distillation sous une pression réduite de $4.10^3$ pascals.

On obtient une dispersion vésiculaire ayant une concentration pondérale en phase lipidique de 8 %, un diamètre moyen de vésicules de 240 nm, avec un indice de polydispersité de 0,27.

EXEMPLE 3 : Préparation d'une dispersion aqueuse de vésicules à partir de lipides ioniques

On prépare une phase lipidique formulée comme suit :

- Lécithine hydrogénée à 30/35 % de phosphatidylcholine hydrogéné, vendue par la Société "NIKKO" sous la dénomination "LECINOL S 10" 480 g
- Phytostérol polyoxyéthyléné (à 5 moles d'oxyde d'éthylène) vendu par la Société "NIKKO" sous la dénomination "GENEROL 122 E 5" 320 g
- mélange de chlorure de méthylène et de chloroforme dans les proportions 81,5 18,5 en poids 32OO g

On prépare une phase aqueuse formulée comme suit :

- eau 13800 g
- sel disodique de l'acide éthylènediaminotétracétique 10 g.

On dissout la phase lipidique dans le mélange de solvants à une température de 35°C. On obtient ainsi une phase organique.

On additionne la phase organique à la phase aqueuse dans un bac cylindrique de 50 $dm^3$ de volume utile ayant une hauteur de 40 cm et on disperse pendant 10 minutes sous une agitation à 5000 tours/mn avec une turbine donnant une vitesse périphérique en bout de pales de 40 m/s.

On distille, sous la même agitation, le mélange de solvants, vers 40°C à la pression atmosphérique, pendant 55 minutes.

Ensuite, on réalise un entraînement du mélange résiduel de solvants et d'une partie de l'eau, vers 40°C,

pendant 145 minutes en poursuivant la distillation sous une pression réduite de $4.10^3$ pascals.

On obtient une dispersion vésiculaire ayant une concentration pondérale en phase lipidique de 8,1 %, un diamètre moyen de vésicules de 204 nm, avec un indice de polydispersité de 0,3.

## Revendications

**1.** Procédé de préparation d'une dispersion aqueuse de vésicules lipidiques lamellaires, chacune de ces vésicules étant constituée par au moins un feuillet lipidique sphéroïde encapsulant un liquide aqueux, dans lequel :

(a) dans une première étape, on dissout au moins un lipide dans au moins un solvant organique,

(b) dans une deuxième étape, on ajoute la phase organique obtenue à la première étape à une phase aqueuse,

(c) dans une troisième étape, on disperse le mélange de la deuxième étape sous agitation, et

(d) dans une quatrième étape, on évapore le(ou les) solvants(s) en même temps qu'une partie de l'eau, caractérisé par le fait que le(s) solvant(s) organique(s) de l'étape a) est (sont) non miscible(s) à l'eau ; que les quantités respectives des deux phases de l'étape b) sont telles que la dispersion obtenue ultérieurement est une dispersion du type huile-dans-eau ; et que les étapes c) et d) sont menées sous forte agitation, la phase continue de la dispersion restant en permanence la phase aqueuse.

**2.** Procédé selon la revendication 1, caractérisé par le fait qu'à l'étape a), on utilise un (ou des) solvant(s) organique(s), dont le point d'ébullition est inférieur à 50°C à une pression comprise entre $1,5 \times 10^4$ et $10^5$ pascals.

**3.** Procédé selon la revendication 2, caractérisé par le fait qu'on choisit le(ou les) solvant(s) dans le groupe formé par le dichlorométhane, le chloroforme, l'acétate d'éthyle, l'acétate de butyle, le formiate d'éthyle, l'hexane, le cyclohexane, le toluène l'éther de pétrole, et les mélanges d'au moins deux d'entre eux.

**4.** Procédé selon l'une des revendications 1 à 3, caractérisé par le fait qu'à l'étape (a), on prépare une phase organique dans laquelle le(ou les) lipide(s) est (ou sont) présent(s) à raison d'environ 5 à 50% en poids de ladite phase organique.

**5.** Procédé selon l'une des revendications 1 à 4, caractérisé par le fait qu'on conduit l'étape (a) à une température comprise entre 35°C et 55°C.

**6.** Procédé selon l'une des revendications 1 à 5, caractérisé par le fait que l'on choisit le (ou les) lipides (s) parmi les lipides amphiphiles non-ioniques, les lipides amphiphiles ioniques, et les mélanges de lipides non-ionique(s) et ionique(s).

**7.** Procédé selon la revendication 6, caractérisé par le fait qu'on choisit les lipides amphiphiles non-ioniques dans le groupe formé par :

(1) les éthers de polyglycérol, linéaires ou ramifiés, de formule :

$$R\,O\{C_3H_5(OH)O\}_{\overline{n}}\,H$$

dans laquelle :

. $-C_3H_5(OH)O$ est représenté par les structures suivantes prises en mélange ou séparément : $-CH_2CHOHCH_2O-$ ;

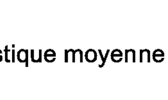 ; 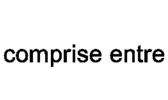 ;

. $\overline{n}$ est une valeur statistique moyenne comprise entre 1 et 6 ;

. R représente :

(a) une chaîne aliphatique, linéaire ou ramifiée, saturée ou insaturée, contenant de 12 à 30 atomes de carbone ; ou des radicaux hydrocarbonés des alcools de lanoline ;

(b) un reste $R^1CO$, où $R^1$ est un radical aliphatique, linéaire ou ramifié, en $C_{11}$-$C_{17}$ ;

(c) un reste $R^2$-[$-OC_2H_3(R^3)$-]-, où :

. $R^2$ peut prendre la signification (a) ou (b) donnée pour R ;

. $OC_2H_3(R^3)$- est représenté par les structures suivantes, prises en mélange ou séparément :

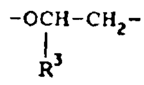 et 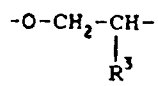

où $R^3$ prend la signification (a) donnée pour R ;

(2) les éthers de polyglycérol, linéaires ou ramifiés, comportant deux chaînes grasses ;

(3) les alcools gras polyoxyéthylénés et les stérols et phytostérols polyoxyéthylénés ;

(4) les éthers de polyols ;

(5) les esters de polyols, oxyéthylénés ou non ;

(6) les glycolipides d'origine naturelle ou synthétique ;

(7) les hydroxyamides représentés par la formule :

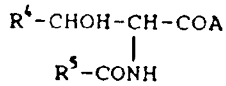

dans laquelle :

- $R^4$ désigne un radical alkyle ou alcényle en $C_7$-$C_{21}$ ;
- $R^5$ désigne un radical hydrocarboné, saturé ou insaturé, en $C_7$-$C_{31}$ ;
- COA désigne un groupement choisi parmi les deux groupements suivants :
  . un reste

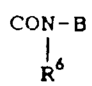

où :

. B est un radical dérivé d'amines primaires ou secondaires, mono- ou polyhydroxylées ; et

. $R^6$ désigne un atome d'hydrogène ou un radical méthyle, éthyle ou hydroxyéthyle ; et

. un reste -COOZ, où Z représente le reste d'un polyol en $C_3$-$C_7$.

**8.** Procédé selon la revendication 6, caractérisé par le fait qu'on choisit les lipides amphiphiles ioniques dans le groupe formé par les phospholipides naturels ou de synthèse, et les composés anioniques.

**9.** Procédé selon l'une des revendications 1 à 8, caractérisé par le fait qu'on associe au(x) lipide(s) au moins un additif choisi dans le groupe formé par :

- les alcools et diols à longue chaîne ;
- les stérols ;
- les amines à longue chaîne et leurs dérivés ammonium-quaternaires ;
- les dihydroxyalkyl-amines ; les amines grasses polyoxyéthylénées ; les esters d'amino-alcools à longue chaîne ; et leurs sels et dérivés ammonium-quaternaires ;
- les esters phosphoriques d'alcools gras ;
- les alkysulfates ;
- certains polypeptides et certaines protéines.

**10.** Procédé selon l'une des revendications 1 à 9, caractérisé par le fait qu'on incorpore au moins une substance active hydrosoluble à la phase aqueuse et/ou au moins une substance active liposoluble à la phase organique.

**11.** Procédé selon l'une des revendications 1 à 10, caractérisé par le fait qu'à l'étape (b), on utilise un rapport pondéral de la phase organique à la phase aqueuse compris entre 0,1 et 0,6.

**12.** Procédé selon l'une des revendications 1 à 11, caractérisé par le fait qu'on conduit les agitations des étapes c) et d) avec des organes d'agitation rotatifs,dont la vitesse périphérique est supérieure à 10 m/s à

l'extrémité libre desdits organes et dont la vitesse angulaire est supérieure à 1000 tours/mn.

**13.** Procédé selon l'une des revendications 1 à 12, caractérisé par le fait qu'on règle la taille des vésicules à une valeur comprise entre 0,2 et 0,3µm par action sur la vitesse d'agitation.

**14.** Procédé selon l'une des revendications 1 à 13, caractérisé par le fait qu'après l'étape (d), on concentre la dispersion.

**15.** Procédé selon la revendication 14, caractérisé par le fait qu'après l'étape (d), on concentre la dispersion par entraînement sous pression réduite du solvant résiduel éventuel et d'une partie de l'eau.

**16.** Composition à base d'une dispersion aqueuse de vésicules obtenue par le procédé selon l'une des revendications 1 à 15.

## Patentansprüche

**1.** Verfahren zur Herstellung einer wäßrigen Dispersion von lamellaren Lipidvesikeln, wobei jedes dieser Vesikel aus wenigstens einem sphäroiden, eine wäßrige Flüssigkeit einkapselnden Lipidplättchen gebildet wird, wobei man:

(a) in einer ersten Stufe wenigstens ein Lipid in wenigstens einem organischen Lösungsmittel löst,
(b) in einer zweiten Stufe die gemäß der ersten Stufe erhaltene organische Phase zu einer wäßrigen Phase gibt,
(c) in einer dritten Stufe die Mischung der zweiten Stufe unter Bewegen dispergiert, und
(d) in einer vierten Stufe das (oder die) Lösungsmittel gleichzeitig mit einem Teil des Wassers verdampft,

**dadurch gekennzeichnet**, daß das organische Lösungsmittel (die organischen Lösungsmittel) der Stufe (a) mit Wasser nicht mischbar ist (sind); daß die jeweiligen Mengen der beiden Phasen der Stufe (b) derart sind, daß die schließlich erhaltene Dispersion eine Dispersion vom Öl-in-Wasser-Typ ist; und daß die Stufen (c) und (d) unter heftigem Bewegen durchgeführt werden, wobei die wäßrige Phase permanent die kontinuierliche Phase der Dispersion bleibt.

**2.** Verfahren nach Anspruch 1, dadurch gekennzeichnet, daß man in der Stufe (a) ein organisches Lösungsmittel (organische Lösungsmittel) verwendet, dessen (deren) Siedepunkt bei einem Druck zwischen 1,5 x $10^4$ und $10^5$ Pascal unterhalb von 50°C liegt.

**3.** Verfahren nach Anspruch 2, dadurch gekennzeichnet, daß das (oder die) Lösungsmittel ausgewählt ist (sind) unter Dichlormethan, Chloroform, Ethylacetat, Butylacetat, Ethylformiat, Hexan, Cyclohexan, Toluol, Petroläther und Mischungen von wenigstens zwei dieser Lösungsmittel.

**4.** Verfahren nach einem der Ansprüche 1 bis 3, dadurch gekennzeichnet, daß man in der Stufe (a) eine organische Phase herstellt, in welcher das Lipid (oder die Lipide) in einer Menge von etwa 5 bis 50 Gew.-% der organischen Phase vorhanden ist (sind).

**5.** Verfahren nach einem der Ansprüche 1 bis 4, dadurch gekennzeichnet, daß man die Stufe (a) bei einer Temperatur zwischen 35°C und 55°C durchführt.

**6.** Verfahren nach einem der Ansprüche 1 bis 5, dadurch gekennzeichnet, daß das Lipid (oder die Lipide) ausgewählt ist (sind) unter nicht-ionischen amphiphilen Lipiden, ionischen amphiphilen Lipiden und Mischungen von (einem) nicht-ionischen und ionischen Lipid(en).

**7.** Verfahren nach Anspruch 6, dadurch gekennzeichnet, daß die nicht-ionischen amphiphilen Lipide ausgewählt sind unter:

(1) linearen oder verzweigten Polyglycerinäthern der Formel:

$$R\,O\{C_3H_5(OH)O\}_{\overline{n}}\,H$$

worin:

. $-C_3H_5(OH)O$ die folgenden, in Kombination oder einzeln vorliegenden Strukturen repräsentiert:
$-CH_2CHOHCH_2O-$;

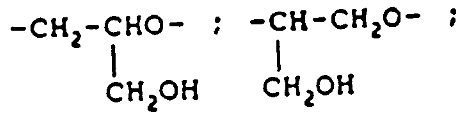

. $\bar{n}$ für einen statistischen Mittelwert zwischen 1 und 6 steht;

. R bedeutet:

(a) eine lineare oder verzweigte, gesättigte oder ungesättigte aliphatische Kette mit 12 bis 30 Kohlenstoffatomen; oder Kohlenwasserstoffreste von Lanolinalkoholen;

(b) einen Rest $R^1CO$, worin $R^1$ einen linearen oder verzweigten, aliphatischen $C_{11}$-$C_{17}$-Rest bedeutet;

(c) einen Rest $R^2$-[-$OC_2H_3(R^3)$-]-, worin:

. $R^2$ die für R angegebenen Bedeutungen (a) oder (b) annehmen kann;

. $OC_2H_3(R^3)$- durch die folgenden in Kombination oder einzeln vorliegenden Strukturen repräsentiert wird:

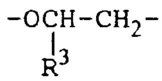 und 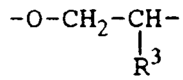

worin $R^3$ die für R angegebenen Bedeutungen (a) besitzt;

(2) linearen oder verzweigten Polyglycerinäthern, die zwei Fettketten aufweisen;

(3) polyoxyethylenieren Fettalkoholen und polyoxyethylenierten Sterolen und Phytosterolen;

(4) Polyoläthern;

(5) gegebenenfalls oxyethylenierten Polyolestern;

(6) Glykolipiden natürlicher oder synthetischer Herkunft;

(7) Hydroxyamiden der Formel:

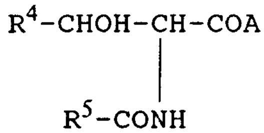

worin:

- $R^6$ einen $C_7$-$C_{21}$-Alkyl- oder -Alkenylrest bedeutet;
- $R^5$ einen gesättigten oder ungesättigten $C_7$-$C_{31}$-Kohlenwasserstoffrest bedeutet;
- COA eine Gruppe bedeutet, die ausgewählt ist unter den beiden folgenden Gruppen:

. einem Rest

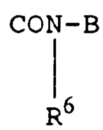

worin:

. B einen von primären oder sekundären, mono-oder polyhydroxylierten Aminen abgeleiteten Rest bedeutet; und

. $R^6$ ein Wasserstoffatom oder einen Methyl-, Ethyl- oder Hydroxyethylrest bedeutet; und

. einem Rest - COOZ, worin Z den Rest eines $C_3$-$C_7$-Polyols bedeutet.

**8.** Verfahren nach Anspruch 6, dadurch gekennzeichnet, daß die ionischen amphiphilen Lipide ausgewählt sind unter natürlichen oder synthetischen Phospholipiden und anionischen Verbindungen.

**9.** Verfahren nach einem der Ansprüche 1 bis 8, dadurch gekennzeichnet, daß man das Lipid (die Lipide) mit wenigstens einem Additiv kombiniert, das ausgewählt ist unter:

- langkettigen Alkoholen und Diolen;
- Sterolen;
- langkettigen Aminen und ihren quaternären Ammoniumderivaten;
- Dihydroxyalkylaminen; polyoxyethylenierten Fettaminen; Estern von Aminoalkoholen mit langer Kette; und ihren Salzen und quaternären Ammoniumderivaten;
- Phosphorestern von Fettalkoholen;
- Alkylsulfaten;
- bestimmten Polypeptiden und bestimmten Proteinen.

**10.** Verfahren nach einem der Ansprüche 1 bis 9, dadurch gekennzeichnet, daß man der wäßrigen Phase wenigstens eine wasserlösliche aktive Substanz und/oder der organischen Phase wenigstens eine fettlösliche aktive Substanz einverleibt.

**11.** Verfahren nach einem der Ansprüche 1 bis 10, dadurch gekennzeichnet, daß in Stufe (b) das Gewichtsverhältnis von organischer Phase zu wäßriger Phase zwischen 0,1 und 0,6 liegt.

**12.** Verfahren nach einem der Ansprüche 1 bis 11, dadurch gekennzeichnet, daß man das Bewegen in den Stufen (c) und (d) mit einer Drehbewegungsvorrichtung durchführt, deren Umfangsgeschwindigkeit größer als 10 m/s am freien Ende der Vorrichtung und deren Winkelgeschwindigkeit größer als 1000 Umdrehungen/min ist.

**13.** Verfahren nach einem der Ansprüche 1 bis 12, dadurch gekennzeichnet, daß man die Größe der Vesikel durch Beeinflussung der Bewegungsgeschwindigkeit auf einen Wert zwischen 0,2 und 0,3 μm einreguliert.

**14.** Verfahren nach einem der Ansprüche 1 bis 13, dadurch gekennzeichnet, daß man nach der Stufe (d) die Dispersion konzentriert.

**15.** Verfahren nach Anspruch 14, dadurch gekennzeichnet, daß man nach der Stufe (d) die Dispersion durch Entfernen von gegebenenfalls noch vorhandenem Lösungsmittel und eines Teils des Wassers unter vermindertem Druck konzentriert.

**16.** Zusammensetzung auf Basis einer nach einem der Ansprüche 1 bis 15 erhältlichen wäßrigen Vesikeldispersion.

## Claims

**1.** Process for the preparation of an aqueous dispersion of lamellar lipid vesicles, each of which is composed of at least one spheroidal lipid sheet which encapsulates an aqueous liquid, in which:
(a) in a first step, at least one lipid is dissolved in at least one organic solvent,
(b) in a second step, the organic phase obtained in the first step is added to an aqueous phase,
(c) in a third step, the mixture from the second step is dispersed with stirring, and
(d) in a fourth step, the solvent(s) is (are) evaporated at the same time as a portion of the water, characterized in that the organic solvent(s) from step (a) is (are) immiscible with water; in that the respective amounts of the two phases from step (b) are such that the dispersion subsequently obtained is a dispersion of the oil-in-water type (O/W); and in that steps (c) and (d) are carried out with vigorous stirring, the continuous phase of the dispersion always remaining the aqueous phase.

**2.** Process according to Claim 1, characterized in that in step (a) an organic solvent(s) is (are) used whose boiling point is below 50°C at a pressure between $1.5 \times 10^4$ and $10^5$ pascals.

**3.** Process according to Claim 2, characterized in that the solvents is(are) selected from the group comprising dichloromethane, chloroform, ethyl acetate, butyl acetate, ethyl formate, hexane, cyclohexane, toluene, petroleum ether, and mixtures of at least two of these with one another.

**4.** Process according to any of Claims 1 to 3, characterized in that in step (a) an organic phase is prepared in which the lipid(s) is (are) present in an amount of about 5 to 50 % by weight of said organic phase.

5. Process according to any of Claims 1 to 4, characterized in that step (a) is carried out at a temperature between 35°C and 55°C.

6. Process according to any of Claims 1 to 5, characterized in that the lipid(s) is (are) selected from nonionic amphiphilic lipids, ionic amphiphilic lipids, and mixtures of nonionic and ionic lipids.

7. Process according to Claim 6, characterized in that the nonionic amphiphilic lipids are selected from the group comprising:
(1) linear or branched polyglycerol ethers of the formula:

$$RO\{C_3H_5(OH)O\}_{\bar{n}}-H$$

in which:
$-C_3H_5(OH)O$ is represented by the following structures in a mixture or separately:
$-CH_2CHOHCH_2O-$;

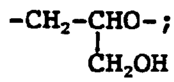 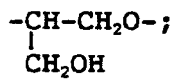

$\bar{n}$ is an average statistic value between 1 and 6;
R represents:
(a) a linear or branched, saturated or unsaturated, aliphatic chain containing 12 to 30 carbon atoms; or hydrocarbon radicals of lanolin alcohols;
(b) a radical $R^1CO$ where $R^1$ is a linear or branched aliphatic $C_{11}$-$C_{17}$ radical
(c) a radical $R_2$-[-$OC_2H_3(R^3)$-]- where:
$R^2$ can take the meaning (a) or (b) given for R;
$OC_2H_3(R^3)$- is represented the following structures in a mixture or separately:

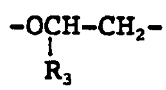 and 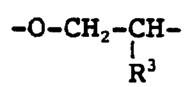

where $R^3$ takes the meaning (a) given for R;
(2) linear or branched polyglycerol ethers comprising two fatty chains;
(3) polyethoxylated fatty alcohols and polyethoxylated sterols and phytosterols;
(4) polyol ethers;
(5) ethoxylated or non-ethoxylated polyol esters;
(6) glycolipids of natural or synthetic origin;
(7) hydroxyamides having the formula:

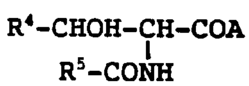

in which:
$R^4$ denotes a $C_7$-$C_{21}$ -alkyl or alkenyl radical;
$R^5$ denotes a saturated or unsaturated $C_7$-$C_{31}$ hydrocarbon radical;
COA denotes a grouping selected from the following two groupings:
a radical

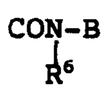

where
B is a radical derived from mono- or polyhydroxylated primary or secondary amines; and
$R^6$ denotes a hydrogen atom or a methyl, ethyl or hydroxyethyl radical; and
a -COOZ radical where Z represents a $C_3$-$C_7$ polyol radical.

**8.** Process according to Claim 6, characterized in that the ionic amphiphilic lipids are selected from the group comprising natural or synthetic phospholipids and anionic compounds.

**9.** Process according to any of Claims 1 to 8, characterized in that the lipid(s) is(are) combined with at least one additive selected from the group comprising:

long-chain alcohols and diols;
sterols;
long-chain amines and quaternary ammonium derivatives thereof;
dihydroxyalkylamines; polyethoxylated fatty amines;
esters of long-chain amino alcohols; and salts and
quaternary ammonium derivatives thereof;
phosphoric esters of fatty alcohols;
alkyl sulphates;
certain polypeptides and certain proteins.

**10.** Process according to any of Claims 1 to 9, characterized in that at least one water-soluble active substance is introduced into the aqueous phase and/or at least one lipid-soluble active substance into the organic phase.

**11.** Process according to any of Claims 1 to 10, characterized in that the weight ratio of the organic phase to the aqueous phase used in step (b) is between 0.1 and 0.6.

**12.** Process according to any of Claims 1 to 11, characterized in that the stirring in steps (c) and (d) is carried out by means of rotating stirrers whose circumferential velocity is greater than 10 m/s at the free end of these devices and whose angular velocity is greater than 1000 rpm.

**13.** Process according to any of Claims 1 to 12, characterized in that the vesicle size is controlled at a value between 0.2 and 0.3 μm by acting on the stirrer velocity.

**14.** Process according to any of Claims 1 to 13, characterized in that after step (d) the dispersion is concentrated.

**15.** Process according to Claim 14, characterized in that after step (d) the dispersion is concentrated by entrainment of any residual solvent and of a portion of the water under reduced pressure.

**16.** Composition based on an aqueous dispersion of vesicles obtained by the process according to any of Claims 1 to 15.

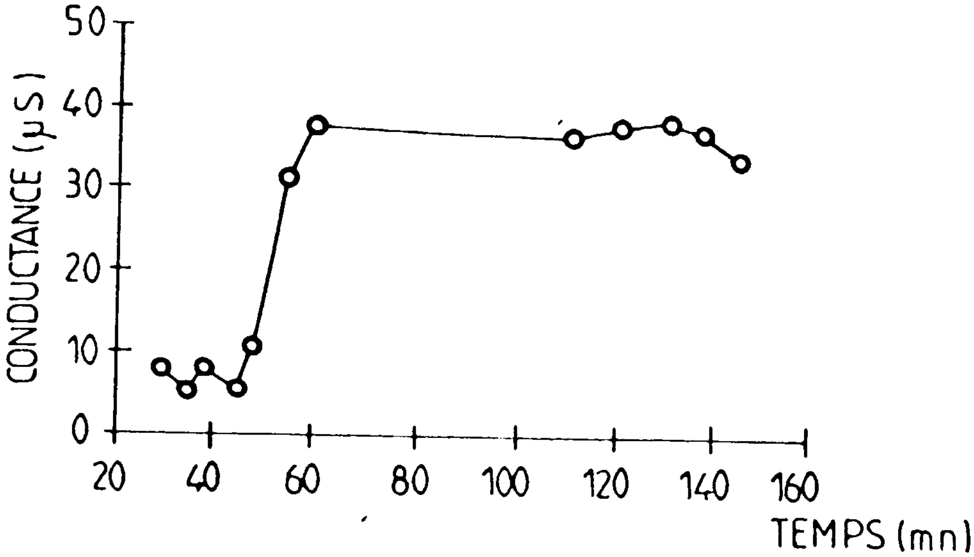
CONDUCTANCE (µS)
50
40
30
20
10
0
20
40
60
80
100
120
140
160
TEMPS (mn)

FIG.1